(19) **Europäisches Patentamt**
**European Patent Office**
**Office européen des brevets**

(11) **EP 3 571 991 A1**

(12) **EUROPEAN PATENT APPLICATION**

(43) Date of publication:
**27.11.2019 Bulletin 2019/48**

(51) Int Cl.:
***A61B 5/11*** (2006.01)

(21) Application number: **18173767.7**

(22) Date of filing: **23.05.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **Joshi, Rohan**
**5656 AE Eindhoven (NL)**

• **Bierling, Bart**
**5656 AE Eindhoven (NL)**
• **DELLIMORE, Kiran Hamilton J.**
**5656 AE Eindhoven (NL)**
• **LONG, Xi**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **MEASURING MOVEMENT IN A SUBJECT**

(57) There is provided an apparatus (100) for measuring movement in a subject. The apparatus (100) comprises a processor (102). The processor (102) is configured to acquire a ballistography, BSG, signal obtained from the subject by a BSG sensor (104). The acquired BSG signal is indicative of movement in the subject. The processor (102) is configured to analyze the acquired BSG signal to identify an index that quantifies a stability of the acquired BSG signal and determine a measure of a quantity of movement in the subject based on the identified index.

Fig. 1

**Description**

FIELD OF THE INVENTION

The disclosure relates to an apparatus and method for measuring movement in a subject.

BACKGROUND OF THE INVENTION

It is valuable to monitor a subject in a variety of situations. For example, it can be particularly valuable to monitor a subject in a clinical setting, such as an infant in a neonatal intensive care unit (NICU). Often the monitoring of a subject includes the monitoring of vital signs, such as heart rate (HR), breathing rate (BR) and oxygen desaturation (SpO2). These vital signs are reflective of the functioning of the autonomic nervous system (ANS) of the subject and are monitored to promptly detect physiological derangements in the subject. As a part of routine clinical care, vital signs monitoring is often complemented by intermittent clinical observations for the early detection of derangements and the diagnosis of diseases. Such observations are important because they can provide information that is not captured in vital signs monitoring, such as muscle tone, skin-pallor and gross body movement, amongst others.

However, it can also be valuable to monitor movement in a subject. In fact, it has long been considered clinically important to monitor body movement or the quality of body movement in a subject. While monitoring vital signs of a subject reflects the functioning of the ANS of the subject, monitoring body movement in a subject is reflective of the functioning of the motor system of the subject. Body movement-related symptoms are also sensitive to multiple clinically significant events including sepsis, seizures, apneic episodes, sleep-wake cycles, etc. For example, a decrease in movement activity over time has been identified as being clinically predictive of sepsis and is often considered to be the dominant clinical sign. With regard to seizures, some types of seizure cannot be identified using electroencephalograph (EEG) monitoring but can be detected through body movement monitoring. Also, apneic episodes and, in particular, obstructive apneas are associated with body movement preceding, during or in response to the apnea itself. Monitoring body movement can also facilitate the tracking of sleep-wake cycles of a subject, which can be used to help synchronize periods of nursing care to minimally disturb the sleep of the subject. This can be particularly valuable in the case of a preterm infant, where minimally disturbed sleep is an important factor in aiding their neurodevelopment.

However, while it is valuable in many situations to monitor movement in a subject, techniques for continuous and reliable monitoring of movement in an infant (and, in particular, a neonate) are currently unavailable. For example, it is often the case that clinical personnel are required to intermittently observe a subject to score movement in the subject but this observation is of insufficient frequency. There is thus a need for automated technology to monitor movement.

In this respect, there exist wearable sensors (e.g. accelerometers) for monitoring movement in a subject. However, wearable sensors have many downsides. In particular, wearable sensors are obtrusive and difficult to position (as they require an optimal anatomical location to be found for sensor placement) and they can result in skin irritation and discomfort for the subject. This can be particularly problematic in the case of vulnerable preterm infants. Another downside to using wearable sensors in the case of infants is that wearable sensors increase the sense of detachment between the infants and their parents.

There also exist camera (or video) based monitoring for monitoring movement in a subject. However, while less-obtrusive, this form of monitoring still faces challenges. For example, the view of the camera may be occluded (e.g. by a blanket covering an incubator in the case of a preterm infant) and/or the camera can suffer from insufficient visibility of the subject, such as due to changes in lighting conditions (e.g. due to a dim or dark room being necessary, especially in the case of a preterm infant) and also light-reflections (e.g. from incubator walls in the case of a preterm infant).

There are also many other challenges that exist for sensor technologies. For example, one of the other challenges includes the requirement for sensitivity to subtle movements in a subject and the possibility of integration within a clinical environment, where a subject may be fragile and vulnerable (e.g. the neonatal environment where a preterm infant can be particularly fragile and vulnerable). Thus, the above-mentioned existing techniques for monitoring movement in a subject have their limitations.

Another technique for monitoring movement in a subject is to use the movement artefacts that often corrupt the routinely monitored vital signs, such as electrocardiograph (ECG), chest impedance (CI), photoplethysmograph (PPG), etc. However, the challenge with this technique is that there are often hardware-level solutions employed that suppress these movement artefacts and it can be difficult or even impossible for digital signal processing to subsequently reverse this suppression for the motion artefacts to be detected in the vital signs.

Another technique that can be used to measure movement in a subject is known as ballistography (BSG). BSG monitors mechanical signals generated due to body movement, such as motion due to breathing and/or the heart beating. BSG monitoring techniques use different types of sensors to monitor movement in a subject, such as pressure sensors, weighing scales, optical sensors, accelerometers and piezoelectric sensors. The techniques can be used for a variety of applications including clinical applications, such as for monitoring cardiac performance, hemodynamic changes, sleep and respiration. For example, BSG is sometimes referred to as ballistocardiography (BCG) where ballistic forces of the heart of a subject are measured, either alone or in combination with other movements in the subject. US 9,907,922 discloses an example of a technique that uses BCG sensors to measure the ballistic forces of the heart.

However, even with BSG available to monitor movement in a subject, it is not currently possible to quantify the movement in a subject.

SUMMARY OF THE INVENTION

As noted above, a limitation with existing techniques is that it is not possible to continuously, reliably and unobtrusively monitor movement in a subject. Also, while movement in a subject can be monitored, it is not possible to quantify the movement. It would thus be valuable to have an apparatus and method that is aimed at addressing at least some of the existing problems.

Therefore, according to a first aspect, there is provided an apparatus for measuring movement in a subject. The apparatus comprises a processor configured to acquire a ballistography, BSG, signal obtained from the subject by a BSG sensor. The acquired BSG signal is indicative of movement in the subject. The processor is also configured to analyze the acquired BSG signal to identify an index that quantifies a stability of the acquired BSG signal and determine a measure of a quantity of movement in the subject based on the identified index.

In some embodiments, the processor may be configured to analyze the acquired BSG signal by the processor being configured to determine a function that describes the acquired BSG signal and identify the index indicative of the stability of the acquired BSG signal based on the determined function. In some embodiments, the processor may be configured to determine the function by being configured to superimpose a plurality of kernels along the acquired BSG signal to determine a density along the acquired BSG signal and determine the function based on the determined density along the acquired BSG signal. In some embodiments, the processor may be configured to identify the index that quantifies the stability of the acquired BSG signal as the maximum value of the determined function. In some embodiments, the function that describes the density along the acquired BSG signal may comprise a function that describes a spectral density along the acquired BSG signal or a probability density along the acquired BSG signal.

In some embodiments, the processor may be configured to apply an energy operator to the acquired BSG signal to determine an energy of the acquired BSG signal. In these embodiments, the determined measure of the quantity of movement in the subject may be further based on the determined energy. In some embodiments, the processor may be configured to determine the energy of the acquired BSG signal as the maximum value of the acquired BSG signal with the energy operator applied.

In some embodiments, the processor may be configured to obtain an analytical signal from the acquired BSG signal. In these embodiments, the analytical signal may comprise the acquired BSG signal as a real part of the analytical signal and a transformation of the BSG signal as an imaginary part of the analytical signal. In some embodiments, the processor may be configured to determine a mean value of a modulus of the obtained analytical signal. In these embodiments, the determined measure of the quantity of movement in the subject may be further based on the determined mean value of the modulus of the obtained analytical signal.

In some embodiments, the processor may be configured to determine a measure of a complexity and/or an entropy of the acquired BSG signal. In these embodiments, the determined measure of the quantity of movement in the subject may be further based on the determined measure of the complexity and/or entropy of the acquired BSG signal. In some embodiments, the measure of the complexity and/or entropy of the acquired BSG signal may comprise any one or more of: a measure of an approximate entropy, ApEn, of the acquired BSG signal; a measure of a Kolmogorov complexity, KC, of the acquired BSG signal; and a measure of a permutation entropy, PeEn, of the acquired BSG signal.

In some embodiments, the processor may be configured to acquire one or more physiological characteristic signals obtained from the subject by at least one physiological characteristic sensor and determine a part of the body of the subject to which the movement in the subject relates based on the one or more acquired physiological characteristic signals. In some embodiments, the one or more acquired physiological characteristic signals may comprise two or more acquired physiological characteristic signals obtained from different parts of the body of the subject.

In some embodiments, the processor may be configured to determine a medical state for the subject, a sleep state for the subject and/or a diagnosis for the subject based on the measure of the quantity of movement in the subject.

According to a second aspect, there is provided a method of operating an apparatus to measure movement in a subject. The method comprises acquiring a ballistography, BSG, signal obtained from the subject by a BSG sensor. The acquired BSG signal is indicative of movement in the subject. The method also comprises analyzing the acquired BSG signal to identify an index that quantifies a stability of the acquired BSG signal and determining a measure of a quantity of movement in the subject based on the identified index.

According to a third aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing techniques are addressed. In particular, according to the above-described aspects and embodiments, it is possible to continuously, reliably and unobtrusively measure movement in a subject through the acquisition and analysis of a BSG signal. Moreover, it is possible to quantify the movement in a subject by way of the above-described aspects and embodiments, which can provide valuable insights into the health and wellbeing of the subject in a manner that is easy to understand. This is possible through the use of an index that quantifies a stability of the acquired BSG signal. The use of this index means that the manner in which the movement in the subject is measured is robust to noise. As such, it can be used in a wide variety of settings including clinical settings. Moreover, the use of the index that quantifies a stability of the acquired BSG signal in the determination of a measure of the quantity of movement in the subject provides accurate and reliable quantification of movement in the subject.

There is thus provided an improved manner in which to measure movement in a subject, which overcomes existing problems.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

> Fig. 1 is a block diagram of an apparatus according to an embodiment;
> Fig. 2 is flow chart illustrating a method according to an embodiment;
> Fig. 3 is flow chart illustrating a method according to another embodiment;
> Figs. 4(a)-(b) are illustrations of signals according to an embodiment;
> Figs. 5(a)-(c) are illustrations of signals according to an embodiment;
> Fig. 6 is an illustration of signals according to an embodiment; and
> Fig. 7 is an illustration of signals according to an embodiment.

DETAILED DESCRIPTION OF EMBODIMENTS

As noted above, there is provided herein an improved manner in which to measure movement in a subject, which overcomes existing problems.

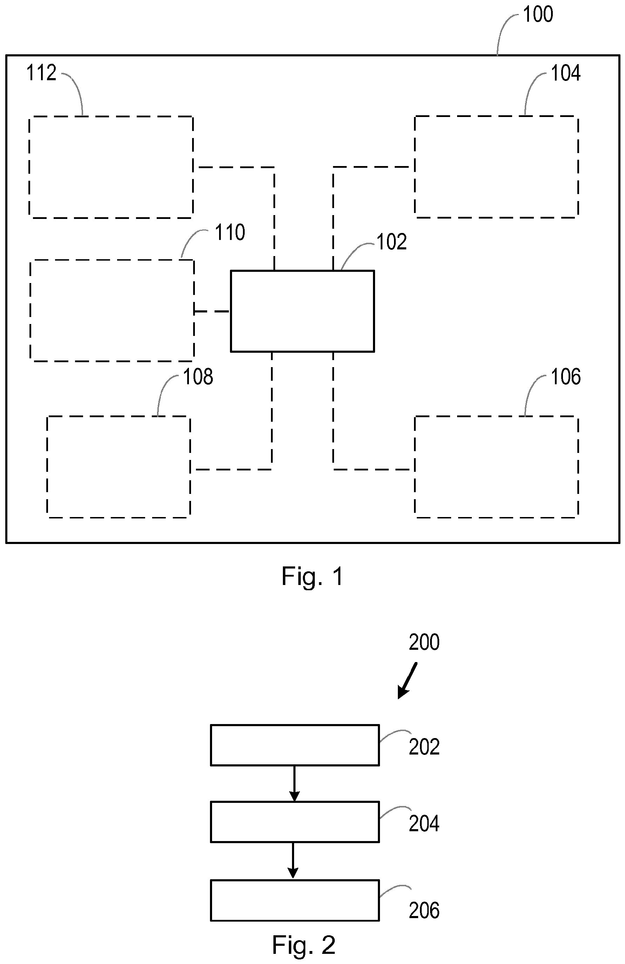

Fig. 1 illustrates an apparatus 100 for measuring movement in a subject according to an embodiment. The subject referred to herein can be, for example, a patient, an animal, or any other subject. In some embodiments, the subject can be an adult. In other embodiments, the subject can be an infant. An infant can, for example, be a neonate (such as a pre-term infant, a full-term infant or a post-term infant) or a child.

As illustrated in Fig. 1, the apparatus 100 comprises a processor 102. Briefly, the processor 102 of the apparatus 100 is configured to acquire a ballistography (BSG) signal. The acquired BSG signal is obtained from the subject by a BSG sensor and is indicative of movement in the subject. The processor 102 of the apparatus 100 is also configured to analyze the acquired BSG signal to identify an index that quantifies a stability of the acquired BSG signal and determine a measure of a quantity of movement in the subject based on the identified index. Herein, the index that quantifies a stability of the acquired BSG signal may also be referred to as a "signal stability index" (SSI) of the acquired BSG signal.

The apparatus 100 may comprise one or more processors 102. The one or more processors 102 can be implemented in numerous ways, with software and/or hardware, to perform the various functions described herein. In some embodiments, each of the one or more processors 102 can be configured to perform individual or multiple steps of the method described herein. In particular implementations, the one or more processors 102 can comprise a plurality of software and/or hardware modules, each configured to perform, or that are for performing, individual or multiple steps of the method described herein. The one or more processors 102 may comprise one or more microprocessors, one or more multi-core processors and/or one or more digital signal processors (DSPs), one or more processing units, and/or one or more controllers (such as one or more microcontrollers) that may be configured or programmed (e.g. using software or computer program code) to perform the various functions described herein. The one or more processors 102 may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

As illustrated in Fig. 1, in some embodiments, the apparatus 100 may comprise the BSG sensor 104. However, it will be understood that, in other embodiments, the BSG sensor 104 may be external to (i.e. separate to or remote from) the apparatus 100. For example, in some embodiments, the BSG sensor 104 can be a separate entity or part of another apparatus (e.g. a device). The BSG sensor 104 is configured to obtain the BSG signal from the subject. The BSG signal obtained from the subject by the BSG sensor 104 is indicative of movement in the subject. The movement in the subject can be any movement in the subject, such as a body movement in the subject, a respiration or breathing related movement in (e.g. the thorax of) the subject, a movement in the subject due to the mechanical action or (micro)motion of the heart (e.g. the BSG sensor 104 may comprise a ballistocardiography (BCG) sensor configured to acquire a BCG signal according to some embodiments), or any other movement in the subject, or any combination of movements in the subject.

The BSG sensor 104 can be any sensor, or any combination of sensors, suitable for obtaining a BSG signal from a subject. Examples of a BSG sensor 104 include, but are not limited to, a pressure sensor, a force sensor, a weight sensor (e.g. a weighing scales), an optical sensor, a motion sensor (e.g. an accelerometer), a piezoelectric sensor, or any other sensor, or any combination of sensors, suitable for obtaining a BSG signal from a subject. In some embodiments, the BSG sensor 104 can comprise a transducer, such as a film-type transducer or thin film-type transducer. In some embodiments, the BSG sensor 104 can be configured to obtain the BSG signal from the subject by being configured to capture movement in the subject in real time. In some embodiments, the BSG sensor 104 can comprise a matrix design. In some of these embodiments, the BSG sensor 104 may also be configured to obtain a spatial location of the movement in the subject.

The BSG sensor 104 can comprise a contact BSG sensor and/or a non-contact BSG sensor. A non-contact BSG sensor can be any BSG sensor 104 that is configured to be remote from (or at a distance from) the subject, such that the BSG sensor 104 has no physical contact with the body of the subject. A contact BSG sensor can be any BSG sensor 104 that is configured to be in physical contact with the body of the subject. In some embodiments, the BSG sensor 104 may be configured to be worn (e.g. against the skin) on a part of the body of the subject (e.g. on a chest, a finger, a wrist and/or any other part of the body of the subject). In some of these embodiments, for example, a band configured to be worn around a part of the body of the subject may comprise the BSG sensor 104 or the BSG sensor 104 may be placed under a band configured to be worn around a part of the body of the subject. In other embodiments, the BSG sensor 104 may be placed inside, on or under a surface on which the subject is positioned. In some of these embodiments, for example, the BSG sensor 104 can be positioned inside, on or under a mattress (e.g. a mattress of a bed, a mattress of an incubator, or any other mattress) or inside, on or under a bed or incubator. In this way, the BSG sensor 104 can be placed underneath the subject in use.

As illustrated in Fig. 1, in some embodiments, the apparatus 100 may comprise a communications interface (or communications circuitry) 106. Alternatively or in addition, the communications interface 106 may be external to (e.g. separate to or remote from) the apparatus 100. The communications interface 106 can be for enabling the apparatus 100, or components of the apparatus 100, to communicate with and/or connect to one or more other components, sensors, interfaces, devices, or memories (such as any of those described herein). For example, the communications interface 106 can be for enabling the processor of the apparatus 100 to communicate with and/or connect to the BSG sensor 104 described earlier. The communications interface 106 may enable the apparatus 100, or components of the apparatus 100, to communicate and/or connect in any suitable way. For example, the communications interface 106 may enable the apparatus 100, or components of the apparatus 100, to communicate and/or connect wirelessly, via a wired connection, or via any other communication (or data transfer) mechanism. In some wireless embodiments, for example, the communications interface 106 may enable the apparatus 100, or components of the apparatus 100, to use radio frequency (RF), Bluetooth, or any other wireless communication technology to communicate and/or connect.

As illustrated in Fig. 1, in some embodiments, the apparatus 100 may comprise a memory 108. Alternatively or in addition, the memory 108 may be external to (e.g. separate to or remote from) the apparatus 100. The processor of the apparatus 100 may be configured to communicate with and/or connect to the memory 108. The memory 108 may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, the memory 108 can be configured to store program code that can be executed by the processor 102 to cause the apparatus 100 to operate in the manner described herein.

Alternatively, or in addition, in some embodiments, the memory 108 can be configured to store information required by or resulting from the method described herein. For example, in some embodiments, the memory 108 may be configured to store any one or more of the BSG signals obtained from the subject by the BSG sensor 104 (e.g. prior to the processor 102 acquiring the signal), the BSG signal acquired by the processor 102 (e.g. where the processor 102 acquires the BSG signal from the BSG sensor 104), the index that quantifies the stability of the acquired BSG signal, the determined measure of the quantity of movement in the subject, or any other information, or any combination of information, required by or resulting from the method described herein. In some embodiments, the processor 102 of the apparatus 100 can be configured to control the memory 108 to store information required by or resulting from the method described herein.

As illustrated in Fig. 1, in some embodiments, the apparatus 100 may comprise a user interface 110. Alternatively or in addition, the user interface 110 may be external to (e.g. separate to or remote from) the apparatus 100. The processor 102 of the apparatus 100 may be configured to communicate with and/or connect to a user interface 110. The user interface 110 can be configured to render (e.g. output, display, or provide) information required by or resulting from the method described herein. For example, in some embodiments, the user interface 110 may be configured to render (e.g. output, display, or provide) one or more of the BSG signal obtained from the subject by the BSG sensor 104 (e.g. prior to the processor 102 acquiring the signal), the BSG signal acquired by the processor 102 (e.g. where the processor 102 acquires the BSG signal from the BSG sensor 104), the index that quantifies the stability of the acquired BSG signal, the determined measure of the quantity of movement in the subject, or any other information, or any combination of information, required by or resulting from the method described herein. Alternatively or in addition, the user interface 110 can be configured to receive a user input. For example, the user interface 110 may allow a user to manually enter information or instructions, interact with and/or control the apparatus 100. Thus, the user interface 110 may be any user interface that enables the rendering (or outputting, displaying, or providing) of information and, alternatively or in addition, enables a user to provide a user input. In some embodiments, the processor 102 of the apparatus 100 can be configured to control the user interface 110 to operate in the manner described herein.

For example, the user interface 110 may comprise one or more switches, one or more buttons, a keypad, a keyboard, a mouse, a touch screen or an application (e.g. on a smart device such as a tablet, a smartphone, smartwatch, or any other smart device), a display or display screen, a graphical user interface (GUI) such as a touch screen, or any other visual component, one or more speakers, one or more microphones or any other audio component, one or more lights (e.g. light emitting diode LED lights), a component for providing tactile or haptic feedback (e.g. a vibration function, or any other tactile feedback component), an augmented reality device (e.g. augmented reality glasses, or any other augmented reality device), a smart device (e.g. a smart mirror, a tablet, a smart phone, a smart watch, or any other smart device), or any other user interface, or combination of user interfaces. In some embodiments, the user interface that is controlled to render information may be the same user interface as that which enables the user to provide a user input.

Although not illustrated in Fig 1, the apparatus 100 may comprise a battery or other power supply for powering the apparatus 100 or means for connecting the apparatus 100 to a mains power supply. It will also be understood that the apparatus 100 may comprise any other component to those described herein or any combination of components.

Fig. 2 illustrates a method 200 of operating an apparatus 100 (as described earlier with reference to Fig. 1) to measure movement in a subject according to an embodiment. As described earlier, the apparatus 100 comprises a processor 102. The method 200 illustrated in Fig. 2 can generally be performed by or under the control of the processor 102. In some embodiments, the method 200 can be referred to as a non-parametric method.

With reference to Fig. 2, at block 202, a BSG signal obtained from the subject by a BSG sensor 104 is acquired. More specifically, as mentioned earlier, the BSG signal is acquired by the processor 102 of the apparatus 100. The acquired BSG signal is indicative of movement in the subject. In some embodiments, the processor 102 can be configured to acquire the BSG signal from the BSG sensor 104 that obtained the BSG signal from the subject. In other embodiments, the processor 102 can be configured to acquire the BSG signal from a memory 108 in which the BSG signal obtained from the subject by the BSG sensor 104 is stored. As mentioned earlier, the memory 108 can be a memory of the apparatus 100 or a memory external to (e.g. separate to or remote from) the apparatus 100.

At block 204 of Fig. 2, the acquired BSG signal is analyzed to identify an index that quantifies a stability of the acquired BSG signal. More specifically, the processor 102 analyzes the acquired BSG signal to identify the index that quantifies the stability of the acquired BSG signal. In some embodiments, the acquired BSG signal may be windowed prior to this analysis. That is, the acquired BSG signal may be segmented (or divided or split) into a plurality of windows. The size of a window may vary, for example, based on the application in respect of which the method 200 is performed. In some embodiments where the acquired BSG signal is windowed, the processor 102 can be configured to analyze the windowed BSG signal.

In some embodiments, the processor 102 may be configured to analyze the acquired BSG signal by the processor 102 being configured to determine a function that describes the acquired BSG signal and identify the index indicative of the stability of the acquired BSG signal based on the determined function. For example, in some embodiments, the processor 102 may be configured to identify the index that quantifies the stability of the acquired BSG signal as the maximum value (or greatest peak) of the determined function. In some embodiments, the function that describes the density along the acquired BSG signal may comprise a function that describes a spectral (or spectrum) density (e.g. a power spectral density) along (in time) the acquired BSG signal. Thus, the function can be a spectral density function according to some embodiments. In other embodiments the function that describes the density along the acquired BSG signal may comprise a function that describes a probability density along the acquired BSG signal. Thus, the function can be a probability density function (PDF) according to some embodiments.

In some embodiments, the processor 102 can be configured to determine the function by being configured to superimpose a plurality of (or multiple) kernels along the acquired BSG signal to determine a density (or a best estimate of the density) along the acquired BSG signal and determine the function based on the determined density along the acquired BSG signal. Thus, according to some embodiments, the processor 102 can be configured to determine a kernel density estimate for the acquired BSG signal and determine the function based on the determined kernel density estimate for the acquired BSG signal. In some of these embodiments, the plurality of kernels may be superimposed along the acquired BSG signal at equal distances from each other. In other words, the plurality of kernels may be superimposed equidistantly along the acquired BSG signal according to some embodiments. In some embodiments, the plurality of kernels can each be superimposed along a predefined region of the BSG signal. In these embodiments, each kernel may be configured to determine a density (or a best estimate of the density) along the predefined region of the BSG signal. For example, the plurality of kernels can each be configured to determine a density (or a best estimate of the density) along the acquired BSG signal in their neighborhood.

The plurality of kernels in these embodiments may comprise any type of kernels or any combination of kernels. Examples of kernels that may be used include, but are not limited to, a Gaussian kernel, a uniform kernel, a triangle kernel a box kernel, an Epanechnikov kernel, a sine kernel, or any other kernel, or any combination of kernels. In some embodiments, the plurality of kernels may have one or more parameters associated with them. For example, the one or more parameters associated with a Gaussian kernel can comprise a mean and a variance. However, it will be understood that other kernels may have other one or more parameters associated with them.

In these embodiments, the plurality of kernels can be configured to change one or more of their parameters to allow the determination of the density along the acquired signal. Thus, for example, a plurality of Gaussian kernels can be configured to change their mean and variance to allow the determination of the density along the acquired signal. In this example, if the acquired BSG signal is regular, then the mean and variance of the plurality of Gaussian kernels is similar. As such, superimposing this plurality of Gaussian kernels along the acquired BSG signal results in a narrow function with a high peak. On the other hand, if the acquired BSG signal is irregular, then the mean and variance of the plurality of Gaussian kernels are different. As such, superimposing this plurality of Gaussian kernels along the acquired BSG signal results in a broad function with limited peaks.

Thus, returning to Fig. 2, an index that quantifies a stability of the acquired BSG signal is identified at block 204 of Fig. 2. At block 206 of Fig. 2, a measure of a quantity of movement in the subject is determined based on the identified index. In some embodiments, for example, the identified index may be plotted over time and the measure of the quantity of movement in the subject may then be determined as the area under the plot.

Although not illustrated in Fig. 2, in some embodiments, the measure of the quantity of movement in the subject may be determined based on other features in addition to the identified index. For example, in addition to the algorithm involving the signal stability index (SSI) of the acquired BSG signal, one or more other algorithms may also be used in measuring movement in the subject or, more specifically, in quantifying the measurement.

For example, in some embodiments, the processor 102 may be configured to apply an energy operator to the acquired BSG signal to determine an energy of the acquired BSG signal and the determined measure of the quantity of movement in the subject may then further be based on the determined energy. Examples of an energy operator that may be applied to the acquired BSG signal include, but are not limited to, a non-liner operator such as a Teager energy operator (TEO), a spectral energy operator, a fractal dimension operator (e.g. a Higuchi algorithm, a Katz algorithm, a Hurst exponent, or any other fractal dimension operator, or any combination thereof), or any other energy operator, or any combination of energy operators.

In some of these embodiments, the processor 102 may be configured to determine the energy of the acquired BSG signal as the maximum value of the acquired BSG signal with the energy operator applied. In some embodiments, the energy operator can be configured to determine the energy of the acquired BSG signal in a (e.g. small) predefined time window of the acquired BSG signal. In this way, the method can be optimized for local time analysis. In some embodiments, the processor 102 may be configured to determine the energy of the acquired BSG signal at certain time intervals (e.g. every second). Alternatively or in addition, in some embodiments, the processor 102 may be configured to determine the energy of the acquired BSG signal at a plurality (e.g. every) data point on the acquired BSG signal or at a plurality (e.g. every) data point on a window of the acquired BSG signal. In this way, a plurality of energies along the acquired BSG signal can be determined. In some of these embodiments, the processor 102 may be configured to determine the energy of the acquired BSG signal as the maximum value of these determined energies.

Alternatively, or in addition, in some embodiments, the processor 102 can be configured to obtain an analytical signal from the acquired BSG signal and determine a mean value of a modulus of the obtained analytical signal. In these embodiments, the determined measure of the quantity of movement in the subject may then further be based on the determined mean value of the modulus of the obtained analytical signal. In some embodiments, the analytical signal can comprise a real part of the analytical signal and an imaginary part of the analytical signal. For example, in some embodiments, the analytical signal may comprise the acquired BSG signal as a real part of the analytical signal and a transformation of the acquired BSG signal as an imaginary part of the analytical signal. Thus, in some embodiments, the analytical signal $z(t)$ can be defined as a complex signal that comprises the originally acquired BSG signal $x(t)$

and its transform h(t), as follows:

$$z(t) = x(t) + jh(t).$$

Examples of a transformation that may be applied to the acquired BSG signal include, but are not limited to, a Hilbert transformation (HT), a Fourier transform, a wavelet transform, or any other transformation, or any combination of transformations. In some embodiments, the transform (such as the HT transform) of the acquired BSG signal can be described as the envelope of the acquired BSG signal. Thus, the transform can be used for envelope detection according to some embodiments. In embodiments where the acquired BSG signal is windowed, the transform may be used for local envelope detection. The envelope of the acquired BSG signal can be a proxy for (e.g. represent) the energy of the acquired BSG signal according to some embodiments. In other embodiments, the transform (such as the Fourier transform or the wavelet transform) of the acquired BSG signal can be pre-processed to determine the energy of the acquired BSG signal.

Alternatively or in addition, in some embodiments, the processor 102 may be configured to determine a measure of a complexity of the acquired BSG signal. In these embodiments, the determined measure of the quantity of movement in the subject may further be based on the determined measure of the complexity of the acquired BSG signal. Alternatively or in addition, in some embodiments, the processor 102 may be configured to determine a measure of an entropy of the acquired BSG signal. In these embodiments, the determined measure of the quantity of movement in the subject may further be based on the determined measure of the entropy of the acquired BSG signal. Thus, in some embodiments, the determined measure of the quantity of movement in the subject may further be based on any one or more of the determined measure of the complexity and/or the determined measure of the entropy of the acquired BSG signal.

Examples of the measure of the complexity of the acquired BSG signal comprise, but are not limited to, any one or more of a measure of a Kolmogorov complexity (KC) of the acquired BSG signal, a minimum set of Markov states acting as a proxy for (e.g. representing) a measure of a complexity of the acquired BSG signal, or any other measure of the complexity of the acquired BSG signal, or any combination of measures of the complexity of the acquired BSG signal.

Examples of the measure of the entropy of the acquired BSG signal comprise, but are not limited to, any one or more of a measure of an approximate entropy (ApEn) of the acquired BSG signal, a measure of a permutation entropy (PeEn) of the acquired BSG signal, a measure of a Fourier entropy of the acquired BSG signal, a measure of a sample entropy of the acquired BSG signal, a measure of a wavelet entropy of the acquired BSG signal, a measure of a Renyi entropy of the acquired BSG signal, a measure of a Lyapunov exponent of the acquired BSG signal, a measure of a Shannon entropy of the acquired BSG signal, or any other measure of the entropy of the acquired BSG signal, or any combination of measures of the entropy of the acquired BSG signal.

The ApEn of the acquired BSG signal can be defined as a regularity statistic. For example, in some embodiments, the ApEn of the acquired BSG signal can be used to quantify the unpredictability of the time-series data of the acquired BSG signal. In this respect, where the time-series data of the acquired BSG signal is regular or predictable (e.g. comprises repetitive patterns), the ApEn is lower than where the time-series data of the acquired BSG signal is irregular or unpredictable (e.g. comprises no repetitive patterns). On the other hand, where the time-series data of the acquired BSG signal is irregular or unpredictable (e.g. comprises no repetitive patterns), the ApEn is higher than where the time-series data of the acquired BSG signal is regular or predictable (e.g. comprises repetitive patterns). The PeEn of the acquired BSG signal can be defined as a complexity measure. In some embodiments, the PeEn may take into account a temporal order of the data of the acquired BSG signal, for example, by using symbolic dynamics.

Although not illustrated in Fig. 2, in some embodiments, the processor 102 may be configured to acquire one or more physiological characteristic signals (e.g. one or more vital signs such as oxygen saturation). The one or more physiological characteristic signals may be obtained from the subject by at least one physiological characteristic sensor (e.g. at least one vital signs sensor). The at least one physiological characteristic sensor can be configured to obtain the physiological characteristic signal from the subject. In some embodiments, the processor 102 can be configured to acquire one or more physiological characteristic signals from at least one physiological characteristic sensor that obtained the one or more physiological characteristic signals from the subject. Alternatively or in addition, in some embodiments, the processor 102 can be configured to acquire one or more physiological characteristic signals from a memory 108 in which the one or more physiological characteristic signals obtained from the subject by the at least one physiological characteristic sensor is stored. As mentioned earlier, the memory 108 can be a memory of the apparatus 100 or a memory external to (e.g. separate to or remote from) the apparatus 100.

Although not illustrated in Fig. 1, in some embodiments, the apparatus 100 may comprise at least one physiological characteristic sensor. Alternatively or in addition, in some embodiments, at least one physiological characteristic sensor may be external to (i.e. separate to or remote from) the apparatus 100. For example, in some embodiments, at least one physiological characteristic sensor can be a separate entity or part of another apparatus (e.g. a device). The at least one physiological characteristic sensor can be any sensor, or any combination of sensors, suitable for obtaining one or more physiological characteristic signals from a subject. Examples of a physiological characteristic sensor include, but are not limited to, an electrocardiograph (ECG) sensor, a chest impedance (CI) sensor, a photoplethysmograph (PPG) sensor (such as a remote PPG (rPPG) sensor, an image PPG (iPPG) sensor, or any other PPG sensor), a respiratory inductance plethysmography sensor, a Doppler radar sensor, a pulse oximeter, a vital signs camera, or any other sensor, or any combination of sensors, suitable for obtaining one or more physiological characteristic signals from a subject.

In embodiments where the processor 102 is configured to acquire one or more physiological characteristic signals, the processor 102 may also be configured to determine a part of the body of the subject to which the movement in the subject relates based on the one or more acquired physiological characteristic signals. For example, an acquired physiological characteristic signal obtained from a physiological characteristic signal placed on a particular part of the body of the subject (e.g. a foot) can be used to identify whether that particular part of the body of the subject is moving and thus whether the movement in the subject relates to that particular part of the body of the subject. The movement in the subject may relate to any part of the body of the subject, such as a foot of the subject, the chest of the subject, an arm of the subject, or any other part of the body of the subject.

In some embodiments, the one or more acquired physiological characteristic signals can comprise two or more acquired physiological characteristic signals obtained from different parts of the body of the subject. In some of these embodiments, the processor 102 may be configured to determine a part of the body of the subject to which the movement in the subject is restricted based on the two or more acquired physiological characteristic signals (and optionally also based on the acquired BSG signal). More specifically, the processor 102 can be configured to process the acquired physiological characteristic signals (e.g. in combination with the acquired BSG signal) to determine a part of the body of the subject to which the movement in the subject is restricted.

In some of these embodiments, a first physiological characteristic sensor may be positioned on a first part of the body of the subject and a second physiological characteristic sensor may be positioned on a second part of the body of the subject, where the first and second parts of the body are different. For example, the first part of the body may comprise the thorax of the subject and the second part of the body may comprise a foot of the subject. The first and second physiological characteristic sensor may also be different. For example, the first physiological characteristic sensor may comprise an ECG sensor and the second physiological characteristic sensor may comprise a PPG sensor. Generally, an ECG sensor is more sensitive to upper body movement, while a PPG sensor is more sensitive to lower body movement.

Thus, the processor 102 can be configured to process this information (e.g. in combination with the acquired BSG signal) to determine a part of the body of the subject to which the movement is restricted, such as whether the movement is restricted to the upper body of the subject, the lower body of the subject or comprises movement of the whole body of the subject.

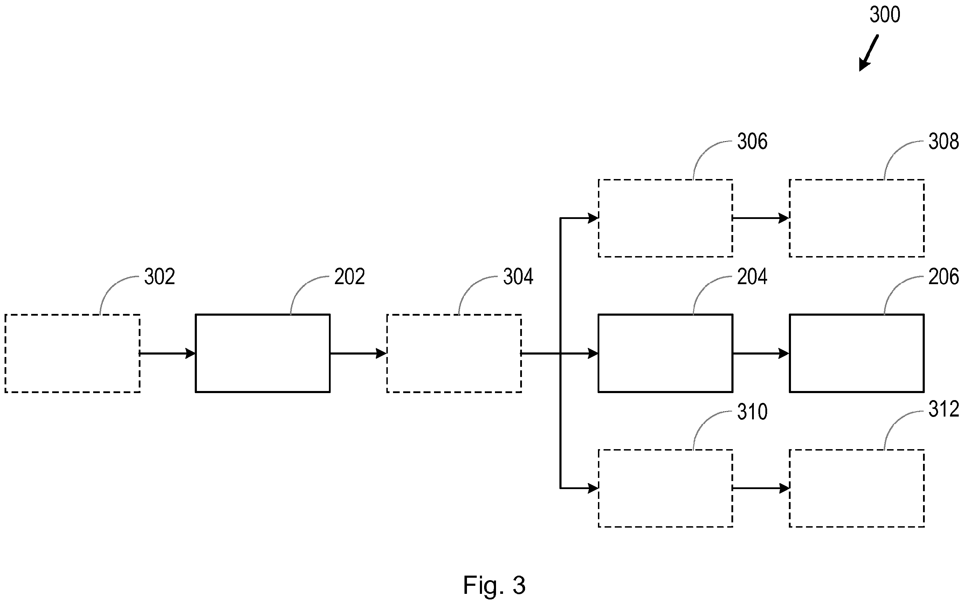

Fig. 3 illustrates a method 300 of operating an apparatus 100 (as described earlier with reference to Fig. 1) to measure movement in a subject according to another embodiment. As described earlier, the apparatus 100 comprises a processor 102. The method 300 illustrated in Fig. 3 can generally be performed by or under the control of the processor 102. In some embodiments, the method 300 can be referred to as a non-parametric method.

With reference to Fig. 3, at block 302 of Fig. 3, a BSG signal is obtained from the subject by a BSG sensor 104 in the manner described earlier. At block 202 of Fig. 3, the BSG signal obtained from the subject by a BSG sensor 104 is acquired by the processor 102 (in the manner described earlier with respect to block 202 of Fig. 2).

At block 304 of Fig. 3, in some embodiments, the acquired BSG signal may filtered and/or amplified by the processor 102. In some embodiments, different frequencies of the acquired BSG signal may be filtered by the processor 102 to filter different types of movement in the subject. For example, heart-beat related movement may be associated with high frequencies, breathing-related movement may be associated with medium frequencies, and body movement may be associated with low frequencies. In some embodiments, the acquired BSG signal may be filtered by the processor 102 using an anti-aliasing filter. In some embodiments, the acquired BSG signal may be filtered by the processor 102 using a band-pass filter, such as a second-order band-pass filter.

In some embodiments, the filtering may be performed with a lower cutoff frequency and a higher cutoff frequency. For example, the filtering may be performed with cutoff frequencies of 0.001 Hz and 0.40 Hz. The lower of the cutoff frequencies can eliminate low-frequency baseline drifts that may occur in the acquired BSG signal over time, while the upper cutoff frequency can capture any movement information in the acquired BSG signal. In some embodiments, the acquired BSG signal may be digitized by the processor 102, e.g. subsequent to filtering the acquired BSG signal.

At block 204 of Fig. 3, the acquired BSG signal is analyzed by the processor 102 to identify an index that quantifies a stability of the acquired BSG signal (in the manner described earlier with reference to block 204 of Fig. 2). At block 206 of Fig. 3, a measure of a quantity of movement in the subject is determined by the processor 102 based on the identified index (in the manner described earlier with reference to block 204 of Fig. 2).

Optionally, as illustrated by blocks 306 and 310 of Fig. 3, one or more other acquired signals (e.g. one or more acquired physiological characteristic signals) may be analyzed by the processor 102 in some embodiments. In these embodiments, as illustrated by blocks 308 and 312 of Fig. 3, one or more other measures (e.g. measures of one or more physiological characteristics) may be determined by the processor 102 from that analysis. For example, at block 306 of Fig. 3, a physiological characteristic signal indicative of a heart rate of the subject may be analyzed by the processor 102 and, at block 308 of Fig. 3, a measure of a heart rate of the subject may be determined by the processor 102. Similarly, for example, at block 310 of Fig. 3, a physiological characteristic signal indicative of a breathing rate of the subject may be analyzed by the processor 102 and, at block 312 of Fig. 3, a measure of a breathing rate of the subject may be determined by the processor 102. A person skilled in the art will be aware of various algorithms that can be used by the processor 102 to determine a measure of a physiological characteristic, such as a measure of a heart rate and/or a measure of a breathing rate.

In some embodiments, the processor 102 may be configured to compare the one or more other determined measures (e.g. the determined measures of one or more physiological characteristics) to the determined measure of a quantity of movement in the subject. In this way, the determined measure of the quantity of movement in the subject can be put into context such that more valuable information can be derived. For example, an increase in the determined measure of the quantity of movement in the subject corresponding to an increase in a measure of the heart rate of the subject may be indicative of an arousal state of the subject during a sleep cycle.

Although not illustrated in Figs. 2 or 3, in some embodiments, the processor 102 can be configured to track a duration and/or intensity of the movement in the subject. Alternatively or in addition, in some embodiments, the processor 102 can be configured to compare the BSG signal obtained by the BSG sensor to one or more signals indicative of movement in a subject obtained by one or more other sensors.

Although also not illustrated in Figs. 2 or 3, in any of the embodiments described herein, the processor 102 may be configured to determine any one or more of a medical state for the subject (e.g. whether the subject is having a seizure, or is in any other medical state), a sleep state for the subject (e.g. a sleep-wake cycle, or any other sleep state) and a diagnosis for the subject (e.g. whether the subject has an apnea, sepsis, or any other diagnosis) based on the measure of the quantity of movement in the subject.

In the case of apneas, there are generally three types of apneas, which include central apnea, obstructive apnea and mixed (i.e. central and obstructive) apnea. There is no respiratory effort in central apnea, whereas respiratory effort is present in obstructive apnea. Thus, obstructive apneas are characterized by the presence of respiratory effort but also by struggling and thrashing movements of the subject. As such, the method disclosed herein can be used to improve identification and classification of apneas. For example, the method disclosed herein can be used to discriminate central apneas from obstructive or mixed apneas. Through improved classification of the type of apnea in this way, a subject can be provided with more appropriate treatment. For example, central apnea is typically treated with caffeine or theophylline, while obstructive apnea is typically managed with increasing respiratory support, such as continuous positive airway pressure (CPAP).

The determination of a measure of a quantity of movement (or the quantification of movement) in a subject in the manner described herein can also be useful when performing cardiopulmonary resuscitation (CPR). For example, when CPR is administered to a subject, the frequency and depth of compressions may be undesirably variable. The method described herein can thus be used to provide feedback to a clinician on the frequency of compressions and whether the force of compression (which is proxy for depth) is varying based on the measure of the quantity of movement in the subject. In some embodiments, an energy expenditure of the subject may be determined by comparing the measure of the quantity of movement in the subject to corresponding measures that are stored in a memory 108 with associated energy expenditure values. The energy expenditure of the subject can be helpful, for example, in ensuring an infant is receiving adequate nutrition during feedings, e.g. by adjusting feeding frequency, feeding amount, or nutritional content. In some embodiments, a synergy between the determined measure of the quantity of movement in the subject and one or more physiological characteristics can be analyzed longitudinally over time, e.g. to study maturation of the subject. For example, this synergy increases with maturity. In some embodiments, time periods having an increase in the determined measure of the quantity of movement (e.g. movement bursts) in the subject can be useful in polysomnography studies.

In some embodiments, the movement in the subject can be used to set a delay on an alarm, such as an alarm of a monitoring device (e.g. a patient monitor). In this way, false alarms as a result of movement artefacts can be avoided. For example, alarms usually have a delay (e.g. a short delay of around 10-15s) after a pre-set threshold for a physiological characteristic is breached and before the alarm is triggered. In some embodiments, the delay can be adjusted based on whether the breach is associated with changes in the movement in the subject. In some embodiments, in the case of infants, an alarm may either be suppressed or communicated in a non-audible format by (e.g. automatically) identifying infant handling. In this way, unnecessary sound pollution can be reduced or even eliminated. For example, infant handling requires a person to be close to an incubator. This can be identified by placing a sensor (e.g. a pressure sensor, an accelerometer, a force sensor, a weight sensor, a BSG sensor, or any other sensor or combination thereof) on or underneath the flooring or a mat in the environment of the incubator. It can thus be determined whether the measure of the quantity of movement in the subject is in fact due to infant handling. The alarm may then be set differently.

Thus, the quantification of movement in the manner described herein can have many useful applications, including clinical applications.

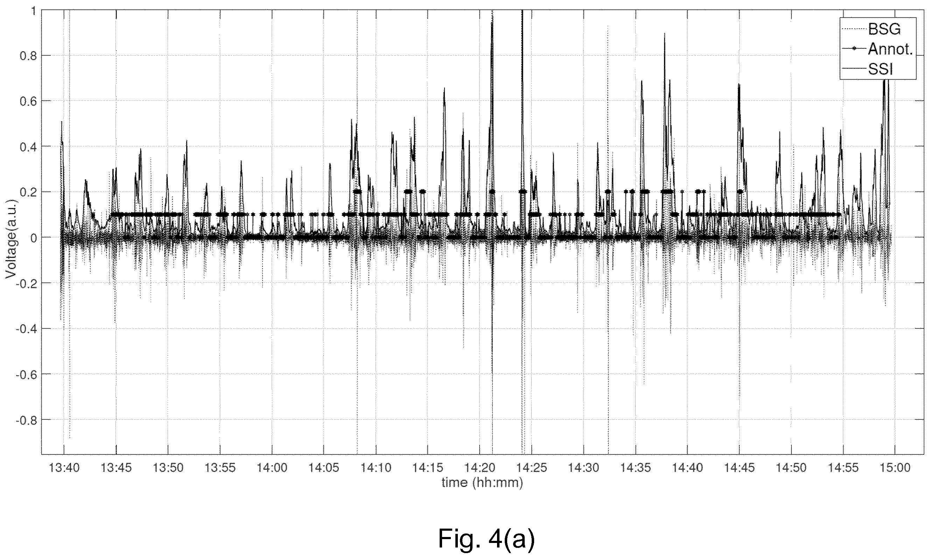

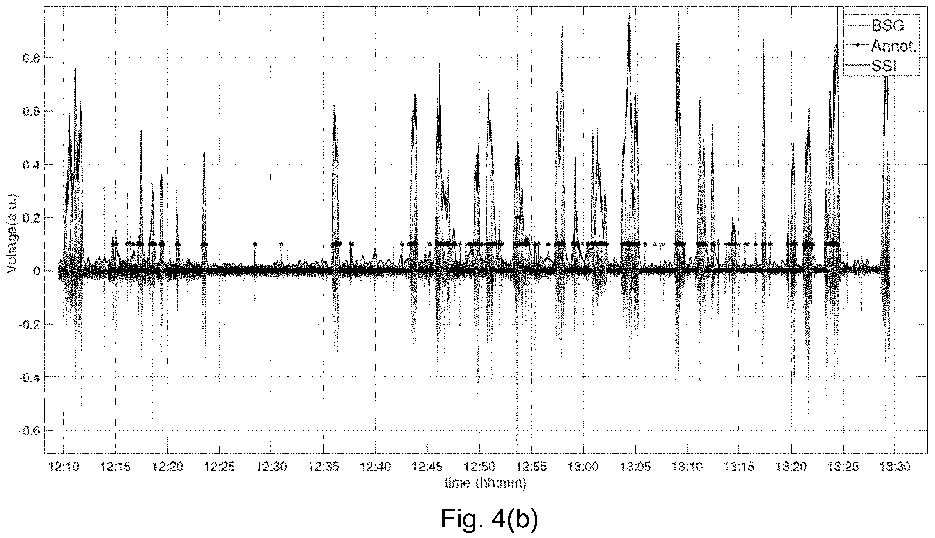

Figs. 4(a) and 4(b) illustrate an acquired BSG signal ("BSG") over time for two different subjects according to an embodiment. The index ("SSI") that quantifies the stability of each of the acquired BSG signals, which is identified in the manner described herein, is also illustrated in Figs. 4(a) and 4(b). The SSI is plotted over time. Also, the illustrated acquired BSG signals for the two subjects are each provided with annotations ("Annot.") that show a measure of the quantity of movement in the subject determined based on the SSI in the manner described herein. The SSI acts as a measure of the quantity of movement in the subject over time. More specifically, in this illustrated embodiment, the measure of the quantity of movement in the subject is determined as the area under the plot of the SSI. Thus, according to this illustrated embodiment, the area under the plot of the SSI acts as the measure of the quantity of movement in the subject.

As illustrated in Figs. 4(a) and 4(b), periodic movement bursts that last about 20-60s, but occasionally up to 2 minutes, can be identified and it can be seen that these movement bursts typically occur every few minutes. At the same time as acquiring the BSG signal, video data may also be acquired on the subject and the time of the identified movement bursts can be found to correspond to large body movements of the head, trunk, and limbs of the subject. Thus, the measure of the quantity of movement in the subject determined in the manner described herein can provide accurate and reliable information on movement in the subject. The acquired BSG signal can provide an overall superior performance due to its ability to capture smaller movements, regardless of whether those smaller movements are localized to a specific body part of the subject.

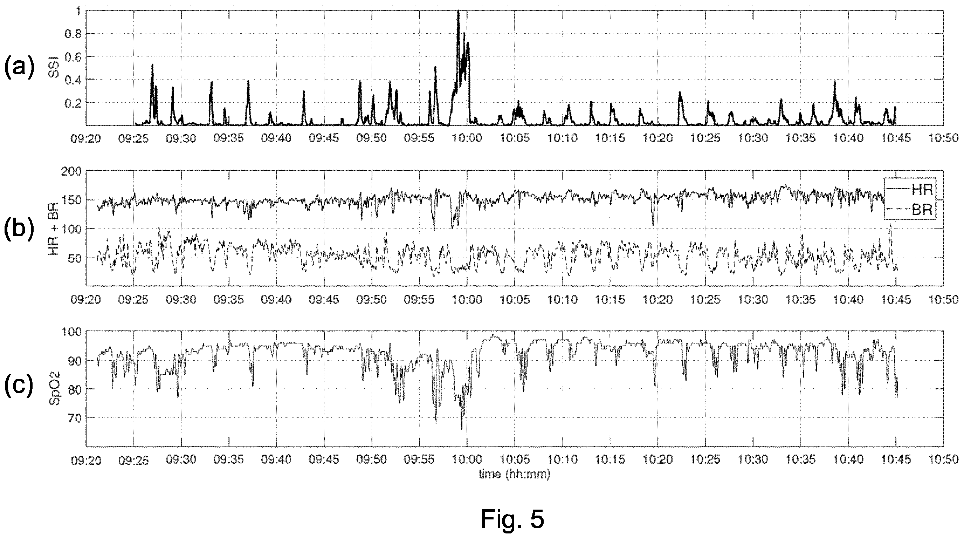

In the illustrated embodiment of Figs. 4(a) and 4(b), the identified movement bursts can be seen to have a repetitive nature, which is indicative of the movement bursts having a physiological origin. For example, the identified movement bursts may be reflective of the arousal state of the subject during a sleep cycle. Similarly, the occasional periods without any movement bursts may be reflective of periods of quiet sleep for the subject (e.g. when the subject is best disturbed for clinical procedures or routine nursing care). This can be confirmed by also analyzing one or more acquired physiological signals obtained from the subject. This is illustrated in Figs. 5(a)-(c).

Fig. 5(a) illustrates an index ("SSI") that quantifies the stability of an acquired BSG signal obtained from a subject, which is identified in the manner described herein. Fig. 5(b) illustrates an acquired heart rate signal ("HR") and an acquired breathing rate signal ("BR") obtained from the subject. Fig. 5(c) illustrates an acquired oxygen desaturation signal ("SpO2") obtained from the subject. Each of the signals obtained from the subject are time synchronized in this illustrated embodiment. As before, the SSI acts as a measure of the quantity of movement in the subject over time. Thus, the increased SSI occurring just before 10:00 in Fig. 5(a) is indicative of an increase in the measure of the quantity of movement in the subject.

As illustrated in Figs. 5(a)-(c), there is a synergy between the increase in the SSI (and thus the movement in the subject) and an increase in the HR of the subject and a decrease in BR of the subject and SpO2 of the subject respectively. This synergy can be indicative of an aroused state of the subject during a sleep cycle. Thus, in some embodiments, the SSI along with physiological characteristics can be used to determine a sleep state of the subject. A correlation coefficient between the SSI and the physiological characteristics (HR, BR and SpO2) can be determined by first standardizing each signal (e.g. subtracting mean and dividing by standard deviation) and determining a normalized correlation coefficient within a time lag of $\pm 30$ seconds, for example, at every data-point, e.g. every second. A person skilled in the art will be aware of the manner in which the correlation coefficient may be determined.

In the illustrated embodiment of Figs. 5 (a)-(c), there is a determined correlation coefficient of 0.40 (0.18) with a time lag of -10.3s (6.9s) between the SSI and HR, a determined correlation coefficient of -0.41 (0.17) with a time lag of -6.6s (4.1s) between the SSI and BR and a determined correlation coefficient of -0.24 (0.13) with a time lag of -14.7s (10s) between the SSI and SpO2. The negative time lags indicate that movement precedes changes in the physiological characteristics (HR, BR and SpO2).

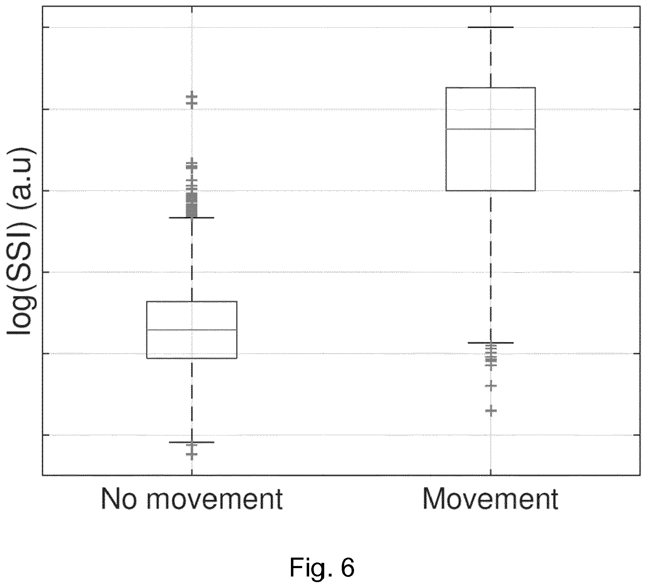

Fig. 6 illustrates a box plot of a logarithm ("log") of the identified index ("SSI") determined form the acquired BSG signal illustrated in Fig. 4(b) in the manner described herein. The logarithm of the identified index is determined form the acquired BSG signal for time-periods corresponding to the categories annotated as "no-movement" in the subject and "movement" in the subject respectively. As illustrated in Fig. 6, the identified index is differentially sensitive to these two annotated categories. That is, the identified index responds differentially to the periods annotated as "no-movement" versus those annotated as a "movement". Thus, it can be seen that the identified index is a reliable feature on which to base the determination of the measure of the quantity of movement in the subject.

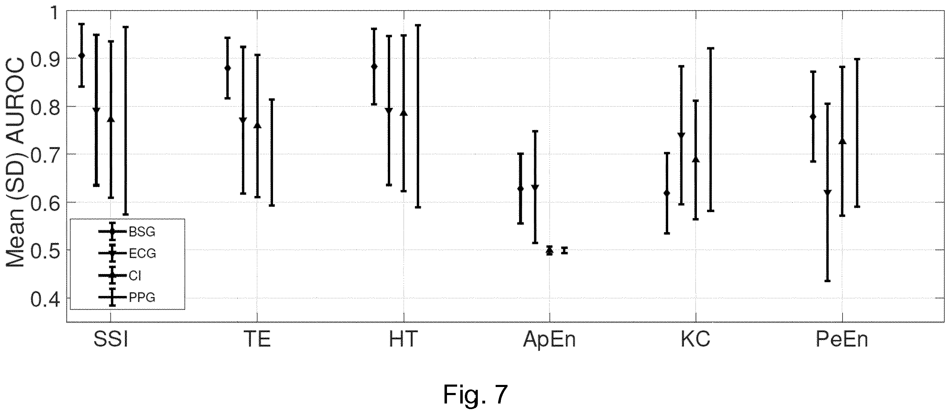

Fig. 7 illustrates a mean or a standard deviation ("SD") of an area under a receiver operating curve (AUROC) for four signals calculated for six features. The AUROC is a measure of the effect size of an ability of a feature to discriminate no-movement of a subject from movement of the subject. The SD of the AUROC shows the improved performance provided through the use of an index ("SSI") that quantifies the stability of an acquired BSG signal obtained from a subject in the determination of a measure of a quantity of movement in a subject as described herein.

In more detail, the four signals illustrated in Fig. 7 comprise the acquired BSG signal ("BSG"), an electrocardiograph signal ("ECG"), a chest impedance signal ("CI"), and a photoplethysomograph signal ("PPG"). The six features for which the SD of the AUROC is determined in Fig. 7 comprise the SSI, a Teager energy ("TE"), a Hilbert transformation ("HT"), an approximate entropy (ApEn), a Kolmogorov complexity (KC), and a permutation entropy (PeEn).

Amongst all features, the best performing features are the SSI and the HT, which are comparable to each other and outperform other features for all signals. The best-performing feature for both the BSG and ECG signals is the SSI with a mean (or standard deviation) AUROC of 0.90 (0.06) and 0.79 (0.15), respectively. The best-performing feature for the CI and the PPG signals is the HT with a mean AUROC of 0.78 (0.15) and 0.77 (0.18) respectively. Amongst all signals, the BSG signal is the best performing for all six features. It can be seen from Fig. 7 that the SD of the AUROC corresponding to the BSG signal, irrespective of the feature under consideration, is considerably smaller than the SD of AUROCs corresponding to other signals, indicating repeatability in monitoring movement compared to other signals. Thus, overall the SSI in combination with the BSG signal provides the most reliable results. The SSI outperforms the other features overall due to the fact that it can combine aspects of monitoring amplitude and regularity. This is in contrast to TE and HT, which are sensitive to amplitude, and ApEn, KC and PeEn, which are sensitive to signal regularity.

In addition to the apparatus 100 and method 200, 300 described earlier, there is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved apparatus, method and computer program product for measuring movement in a subject.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) for measuring movement in a subject, the apparatus (100) comprising a processor (102) configured to:

   acquire a ballistography, BSG, signal obtained from the subject by a BSG sensor (104), wherein the acquired BSG signal is indicative of movement in the subject;
   analyze the acquired BSG signal to identify an index that quantifies a stability of the acquired BSG signal; and
   determine a measure of a quantity of movement in the subject based on the identified index.

2. The apparatus (100) as claimed in claim 1, wherein the processor (102) is configured to analyze the acquired BSG signal to:

   determine a function that describes the acquired BSG in time signal; and
   identify the index indicative of the stability of the acquired BSG signal based on the determined function.

3. The apparatus (100) as claimed in claim 2, wherein the processor (102) is configured to determine the function by being configured to:

superimpose a plurality of kernels along the acquired BSG signal to determine a density along the acquired BSG signal; and

determine the function based on the determined density along the acquired BSG signal.

4. The apparatus (100) as claimed in any of claims 2 or 3, wherein the processor (102) is configured to: identify the index that quantifies the stability of the acquired BSG signal as the maximum value of the determined function.

5. The apparatus (100) as claimed in any of claims 2, 3 or 4, wherein the function that describes the density along the acquired BSG signal comprises a function that describes:

a spectral density along the acquired BSG signal; or

a probability density along the acquired BSG signal.

6. The apparatus (100) as claimed in any of the preceding claims, wherein the processor (102) is configured to:

apply an energy operator to the acquired BSG signal to determine an energy of the acquired BSG signal,

wherein the determined measure of the quantity of movement in the subject is further based on the determined energy.

7. The apparatus (100) as claimed in claim 6, wherein the processor (102) is configured to: determine the energy of the acquired BSG signal as the maximum value of the acquired BSG signal with the energy operator applied.

8. The apparatus (100) as claimed in any of the preceding claims, wherein the processor (102) is configured to:

obtain an analytical signal from the acquired BSG signal, wherein the analytical signal comprises the acquired BSG signal as a real part of the analytical signal and a transformation of the BSG signal as an imaginary part of the analytical signal; and

determine a mean value of a modulus of the obtained analytical signal,

wherein the determined measure of the quantity of movement in the subject is further based on the determined mean value of the modulus of the obtained analytical signal.

9. The apparatus (100) as claimed in any of the preceding claims, wherein the processor (102) is configured to:

determine a measure of a complexity and/or an entropy of the acquired BSG signal,

wherein the determined measure of the quantity of movement in the subject is further based on the determined measure of the complexity and/or entropy of the acquired BSG signal.

10. The apparatus (100) as claimed in claim 9, wherein the measure of the complexity and/or entropy of the acquired BSG signal comprises any one or more of:

a measure of an approximate entropy, ApEn, of the acquired BSG signal;

a measure of a Kolmogorov complexity, KC, of the acquired BSG signal; and

a measure of a permutation entropy, PeEn, of the acquired BSG signal.

11. The apparatus (100) as claimed in any of the preceding claims, wherein the processor (102) is configured to:

acquire one or more physiological characteristic signals obtained from the subject by at least one physiological characteristic sensor; and

determine a part of the body of the subject to which the movement in the subject relates based on the one or more acquired physiological characteristic signals.

12. The apparatus (100) as claimed in claim 11, wherein the one or more acquired physiological characteristic signals comprise two or more acquired physiological characteristic signals obtained from different parts of the body of the subject.

13. The apparatus (100) as claimed in any of the preceding claims, wherein the processor (102) is configured to: determine a medical state for the subject, a sleep state for the subject and/or a diagnosis for the subject based on the measure of the quantity of movement in the subject.

14. A method (200, 300) of operating an apparatus (100) to measure movement in a subject, the method (200, 300) comprising:

acquiring (202) a ballistography, BSG, signal obtained from the subject by a BSG sensor (104), wherein the acquired BSG signal is indicative of movement in the subject;

analyzing (204) the acquired BSG signal to identify an index that quantifies a stability of the acquired BSG signal; and

determining (206) a measure of a quantity of movement in the subject based on the identified index.

15. A computer program product comprising a computer readable medium, the computer readable medium having computer readable code embodied therein, the computer readable code being configured such that, on execution by a suitable computer or processor, the computer or processor is caused to perform the method as claimed in claim 14.

100

112

104

110

102

108

106

Fig. 1

200

202

204

206

Fig. 2

Fig. 3

Fig. 4(a)

Fig. 4(b)

Fig. 5

Fig. 6

Fig. 7

Europäisches
Patentamt

European
Patent Office

Office européen
des brevets

# EUROPEAN SEARCH REPORT

Application Number

EP 18 17 3767

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/247910 A1 (KLAPPER DAVID [US]) 1 October 2009 (2009-10-01) * paragraphs [0117] - [0155], [0167] - [0173], [0208] - [0211]; figures 4,8; tables 4,5,6,12,14 * | 1-15 | INV. A61B5/11 |
| X | US 2009/292180 A1 (MIROW SUSAN [US]) 26 November 2009 (2009-11-26) | 1-10, 13-15 | |
| Y | * paragraphs [0058] - [0068], [0171] - [0214], [0219]; claims 41,42; figures 9,13,14,19,20,33 * * figures 13,19,33 * | 10,11 | |
| X | US 2016/278708 A1 (VRAZIC SACHA [FR]) 29 September 2016 (2016-09-29) | 1,2, 13-15 | |
| Y | * paragraphs [0021] - [0068]; figure 1 * | 10,11 | |
| X | US 2014/163343 A1 (HENEGHAN CONOR [IE] ET AL) 12 June 2014 (2014-06-12) * paragraphs [0038] - [0040], [0043], [0052] - [0060], [0065] - [0075], [0096] - [0109]; figures 1-3,6 * | 1,2, 13-15 | |
| X | US 2017/273635 A1 (LI YELEI [US] ET AL) 28 September 2017 (2017-09-28) * paragraphs [0068] - [0090], [0101] - [0102]; figures 7a-7b,12,13a,13b * | 1,2, 13-15 | TECHNICAL FIELDS SEARCHED (IPC) A61B |
| X | US 2010/030119 A1 (MCNAMES JAMES [US] ET AL) 4 February 2010 (2010-02-04) * paragraphs [0021] - [0034], [0040] - [0042]; figures 1,5 * | 1,2, 13-15 | |

The present search report has been drawn up for all claims

| Place of search | Date of completion of the search | Examiner |
|---|---|---|
| Berlin | 14 November 2018 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 571 991 A1**

## ANNEX TO THE EUROPEAN SEARCH REPORT
## ON EUROPEAN PATENT APPLICATION NO.

EP 18 17 3767

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2018

| Patent document cited in search report | | Publication date | Patent family member(s) | | Publication date |
|---|---|---|---|---|---|
| US 2009247910 | A1 | 01-10-2009 | US | 2005234309 A1 | 20-10-2005 |
| | | | US | 2009247910 A1 | 01-10-2009 |
| US 2009292180 | A1 | 26-11-2009 | EP | 2007277 A2 | 31-12-2008 |
| | | | US | 2009292180 A1 | 26-11-2009 |
| | | | WO | 2007123923 A2 | 01-11-2007 |
| US 2016278708 | A1 | 29-09-2016 | DE | 102015104726 B3 | 02-06-2016 |
| | | | JP | 2016187555 A | 04-11-2016 |
| | | | US | 2016278708 A1 | 29-09-2016 |
| US 2014163343 | A1 | 12-06-2014 | AU | 2007256872 A1 | 13-12-2007 |
| | | | CA | 2654095 A1 | 13-12-2007 |
| | | | CN | 101489478 A | 22-07-2009 |
| | | | EP | 2020919 A2 | 11-02-2009 |
| | | | HK | 1135868 A1 | 10-05-2013 |
| | | | JP | 2009538720 A | 12-11-2009 |
| | | | US | 2009203972 A1 | 13-08-2009 |
| | | | US | 2014163343 A1 | 12-06-2014 |
| | | | WO | 2007143535 A2 | 13-12-2007 |
| US 2017273635 | A1 | 28-09-2017 | CN | 107233086 A | 10-10-2017 |
| | | | EP | 3225158 A2 | 04-10-2017 |
| | | | JP | 2017176827 A | 05-10-2017 |
| | | | KR | 20170113252 A | 12-10-2017 |
| | | | TW | 201733522 A | 01-10-2017 |
| | | | US | 2017273635 A1 | 28-09-2017 |
| US 2010030119 | A1 | 04-02-2010 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9907922 B